# EUROPEAN PATENT SPECIFICATION

(11) **EP 4 382 093 B1**
(45) Date of publication and mention of the grant of the patent: **27.08.2025**
(21) Application number: 22386088.3
(22) Date of filing: 05.12.2022
(51) Int. Cl.: A61K 9/10, A61K 31/7068, A61K 47/14, A61K 9/00

(54) **ORAL SUSPENSIONS COMPRISING CAPECITABINE**
ORALE SUSPENSIONEN MIT CAPECITABIN
SUSPENSIONS ORALES COMPRENANT DE LA CAPÉCITABINE

(43) Date of publication of application: 12.06.2024
(73) Proprietor: a Fine House S.A., 19441 Koropi (GR)
(72) Inventor: Liolios, Georgios, 15232 Chalandri Attica (GR); Tziala, Soultana, 19016 Artemida (GR)
(74) Representative: Roukounas, Dimitrios

(56) References cited:
- WO-A1-2021/033144
- WO-A2-2020/039264
- DUDHIPALA NARENDAR ET AL: "Capecitabine lipid nanoparticles for anti-colon cancer activity in 1,2-dimethylhydrazine-induced colon cancer: preparation, cytotoxic, pharmacokinetic, and pathological evaluation", JOURNAL DRUG DEVELOPMENT AND INDUSTRIAL PHARMACY, vol. 44, no. 10, 4 July 2018 (2018-07-04), US, pages 1572 - 1582, XP093041075, ISSN: 0363-9045, DOI: 10.1080/03639045.2018.1445264

## Description

### FIELD OF THE INVENTION

The present invention relates to oral pharmaceutical suspensions comprising as active substance capecitabine.

### BACKGROUND OF THE INVENTION

Capecitabine or 5'-deoxy-5-fluoro-N-[(pentyloxy)-carbonyl]-cytidine was first disclosed in US5472949. Capecitabine is a fluoropyrimidine carbamate having antineoplastic activity. It is an orally administered systemic prodrug of 5'-deoxy-5-fluorouridine (5'-DFUR), which is converted to fluorouracil.

Capecitabine has the following structure.

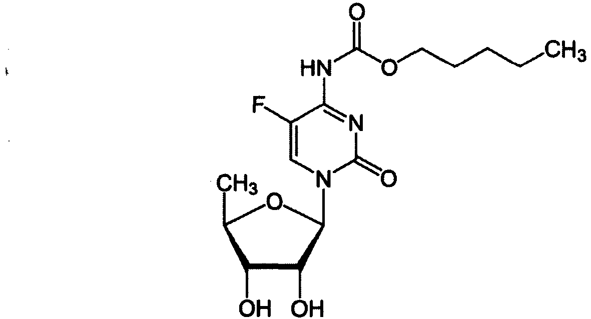

Capecitabine is currently formulated only in solid state forms and is commercially available as 150 mg, 300 mg and 500 mg film coated tablets under the brand name Xeloda^{®} among others.

Although oral solid dosage forms such as tablets are very popular, mainly due to ease of management, for certain users (e.g. children) these forms are not necessarily a convenient option, especially due to difficulty in swallowing these forms. This lack of convenience results in high incidence of non-compliance and ineffective therapy.

Moreover, the Patient Information Leaflet (PIL) of Xeloda^{®} discloses a dosing scheme according to which recommended standard starting dose of capecitabine is 1250 mg/m² (doses are calculated according to body surface area) administered orally twice daily (morning and evening; equivalent to 2500 mg/m² total daily dose) for 2 weeks followed by a 1-week rest period given as 3 -week cycles. In combination with docetaxel, the recommended dose of capecitabine is 1250 mg/m² twice daily for 2 weeks followed by a 1-week rest period, combined with docetaxel at 75 mg/m² as a 1-hour intravenous infusion every 3 weeks.

This dosing scheme sets as prerequisite pharmaceutical forms, such as oral liquid suspensions, that enable dose fractioning.

Drugs are formulated as suspensions for different reasons, but the most common one is low solubility. Suspensions may also be used to mask the poor taste resulting from the dissolved drug in solution.

However, it is apparently extremely challenging to formulate capecitabine in the form of a suspension for oral administration, since it is unstable at least in contact with water. For example, according to M. 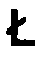 aszcz et. a., "Stability studies of capecitabine", J Therm Anal Calorim (2011) 105:1015-1021, p. 1015 - 1021, capecitabine is not stable even in solid state at 40°C and 75% relative humidity (RH). It has been shown that the main factor in the process of capecitabine degradation is the water sorption, which is privileged at higher temperature and humidity.

Furthermore, a suspension, unlike a syrup in which the drug is fully dissolved, requires adequate shaking of the container to resuspend the drug uniformly before dosing. Difficult redispersion of the drug from a sediment, or in the worst case, from caking, will result in under- and overdosing. This problem of variable dosing is also encountered when the patient or the caregiver forgets to shake the container before dosing. It is therefore desirable to produce a suspension that is able to maintain its homogeneity on prolonged storage without vigorous shaking.

WO2020/039264 discloses oral liquid suspension compositions comprising an anticancer active pharmaceutical ingredient, such as capecitabine, water; a suspending agent, a buffering agent, one or more wetting agents and a binder/filler. The document discloses that a composition comprising capecitabine, xanthan gum, methylparaben, 30% simethicone emulsion, sucralose, tutti frutti flavour, glycerin, citric acid monohydrate, tri-sodium citrate di-hydrate and water exhibits adequate stability at 20°C and 40% RH.

Nevertheless, there is still the need for an oral pharmaceutical suspension, comprising capecitabine, which exhibits excellent physicochemical stability and remain homogeneous during prolonged period of time without vigorous shaking.

### SUMMARY OF INVENTION

The present invention as defined in the claims provides a physiochemically stable oral pharmaceutical suspension comprising capecitabine that remains homogeneous for prolonged period of time without vigorous shaking.

The oral pharmaceutical suspension according to the disclosure comprises capecitabine and a non-aqueous liquid carrier comprising one or more medium-chain fatty acid triglycerides and at least one suspending agent.

The oral pharmaceutical suspension according to the invention exhibits excellent physicochemical stability.

The present invention has the advantage that it also provides an oral pharmaceutical suspension of capecitabine, that remains homogeneous for prolonged period of time without vigorous shaking.

### DETAILED DESCRIPTION OF THE INVENTION

The present invention provides an oral pharmaceutical suspension comprising capecitabine as active ingredient and a non-aqueous liquid carrier comprising one or more triglycerides of saturated fatty acid having a straight chain of 6 to 10 carbon atoms (medium-chain triglycerides) and at least one suspending agent selected form the group consisting of colloidal silicon dioxide, bentonite, potassium alginate, xanthan gum and a mixture of microcrystalline cellulose and sodium carboxymethylcellulose.

The term "% w/v" used in the present description and claims refers to g of the respective substance per 100 ml of the oral suspension.

As used throughout the present description and claims, the term "non-aqueous" means essentially water-free.

As used throughout the present description and claims, the term "suspending agent" means an ingredient that promotes particle suspension or dispersion and reduces sedimentation.

There are numerous lipids comprising triglycerides of fatty acids available to formulators as excipients for lipid-based drug delivery systems. Many synthetic lipids are also available in which the glycerol backbone has been replaced by propylene glycol and/or polyethylene glycols. Additionally, the degree of esterification of the fatty acid moiety may vary, forming mono-, di- and tri-glycerides as well as different esters of propylene glycol and polyethylene glycols. The fatty acids are not necessarily long chain (C₁₁-C₂₂); they can be medium-chain (C₆-C₁₀), short chain, unsaturated or branched. Due to these differences in chemical nature, there are numerous lipids or lipid-like excipients available commercially, all of which are colloquially called 'lipids' in the pharmaceutical field.

The term "medium-chain triglycerides" according to the present invention refers to triglycerides of saturated straight chain fatty acids having a chain of 6 to 10 carbon atoms (C_{6:0} to C_{10:0}).

It has been found that many lipophilic surfactants, commonly used in drug carrier systems such as polysorbate 80, polyethylene glycol castor oils and polyethylene glycol hydrogenated castor oils are ineffective to slow down the decomposition of the active agent after storage.

On the other hand, it has now been found that the physicochemical stability of capecitabine is considerably enhanced in non-aqueous liquid carriers comprising one or more medium-chain fatty acid triglycerides, especially at concentrations of at least 30% w/v. This finding is unexpected since it does not apply to medium-chain fatty acid monoglycerides or diglycerides. In addition, this finding does not apply to other triglycerides, such as long chain triglycerides.

For example, it has been further found that the inclusion of many mixed mono-, or diglycerides, such as glycerol monocaprylocarpate (i.e. medium chain monoglyceride (60% w/w) and diglyceride (35% w/w) consisting of 83% w/w caprylic acid and 17% w/w capric acid) and glycerol dicaprylate (i.e. medium chain diglyceride (83% w/w) comprising 75% - 85% w/w caprylic acid) to oral suspensions comprising capecitabine did not actually enhance the stability of capecitabine at the desired level.

Furthermore, it has been found that the use of certain suspending agents leads to sufficient dispersibility of capecitabine particles in the oral suspension without the need of vigorous shaking of the oral suspension.

The oral pharmaceutical suspension according to the disclosure comprises capecitabine and a non-aqueous liquid carrier comprising one or more medium-chain fatty acid triglycerides and at least one suspending agent selected form the group consisting of colloidal silicon dioxide, bentonite, potassium alginate, xanthan gum and a mixture of microcrystalline cellulose and sodium carboxymethylcellulose. Preferably the suspending agent is selected form the group consisting of colloidal silicon dioxide, potassium alginate and a mixture of microcrystalline cellulose and sodium carboxymethylcellulose. More preferably the suspending agent is colloidal silicon dioxide.

Preferably, the mixture of microcrystalline cellulose and sodium carboxymethylcellulose has 8.3 to 13.8 % w/w sodium carboxymethylcellulose content. Examples of commercially available mixtures of microcrystalline cellulose and sodium carboxymethylcellulose that can be used as suspending agents in the present invention include VIVAPUR^{®} MCG 581 P (8.3 to 13.8 % w/w sodium carboxymethylcellulose content; particle size >250 µm (60 mesh): max. 0.1% and >45 µm (325 mesh): max. 45%; viscosity 72 - 168 mPa.s) and VIVAPUR^{®} MCG 591 P (8.3 to 13.8 % w/w sodium carboxymethylcellulose content; particle size of >250 µm (60 mesh): max. 0.1% and >45 µm (325 mesh): max. 45%; viscosity 39 - 91 mPa.s).

Preferably, the oral pharmaceutical suspension according to the invention comprises from 5.0 % w/v to 40 % w/v capecitabine.

More preferably, the oral pharmaceutical suspension according to the invention comprises from 10 % w/v to 30 % w/v capecitabine.

Preferably, the total concentration of suspending agents in the oral pharmaceutical suspension according to the invention is from 0.1% w/v to 10% w/v. More preferably, the total concentration suspending agents is from 1% w/v to 5% w/v. Even more preferably, the total concentration of suspending agents is from 2% w/v to 4% w/v.

The European Pharmacopoeia (Ph. Eur. 6.0) describes medium-chain triglycerides as the fixed oil extracted from the hard, dried fraction of the endosperm of Cocos nucifera L. or from the dried endosperm of Elaeis guineenis Jacq. They comprise a mixture of triglycerides of saturated fatty acids, mainly of caprylic acid (C_{8:0}) and of capric acid (C_{10:0}). They contain no less than 95% w/w of saturated fatty acids (expressed as percent (%) by weight of total mixture).

According to Handbook of excipients, sixth edition (2009), medium-chain triglycerides (synonyms; MCT oil, caprylic/capric triglyceride, glyceryl tricaprylate/caprate) may also be known as fractionated coconut oil, which contains three saturated lipid chains bound to a glycerin backbone, and are distinguished from other triglycerides by the length of the carbon chains, normally from 6 to 10.

Medium chain triglycerides have been used in a variety of pharmaceutical formulations including oral, parenteral and topical preparations mainly as emulsifying agents or solvents. They are usually colorless to slightly yellow liquids that are practically odorless and tasteless, while they solidify at about 0°C.

According to a preferred embodiment of the invention, the medium-chain triglyceride is caprylic triglyceride.

According to another preferred embodiment of the invention, the medium-chain triglyceride is a mixture of caprylic and capric triglycerides. Preferably, the medium-chain triglyceride according to the invention is a mixture of caprylic and capric triglycerides in a weight ratio caprylic acid : capric acid from 4:6 to 99:1. More preferably, the medium-chain triglyceride according to the invention is a mixture of caprylic and capric triglycerides in a weight ratio caprylic acid : capric acid from 4.5:5.5 to 95:5. Even more preferably, the medium-chain triglyceride according to the invention is a mixture of caprylic and capric triglycerides in a weight ratio caprylic acid : capric acid from 5:5 to 90:10.

Preferably the medium-chain triglyceride according to the invention has a saponification value from 320 mg KOH/g to 380 mg KOH/g. More preferably the medium-chain triglyceride according to the invention has a saponification value from 330 mg KOH/g to 370 mg KOH/g. Even more preferably the medium-chain triglyceride according to the invention has a saponification value from 335 mg KOH/g to 365 mg KOH/g.

The saponification value corresponds to the mass in mg of potassium hydroxide (KOH) needed to neutralize the free fatty acids and saponify the esters contained in a gram of the medium-chain triglyceride material.

Preferable examples of medium-chain triglycerides according to the invention include
∘ caprylic triglyceride with fatty-acid composition of about 99% w/w caprylic acid (C_{8:0}) (synonyms: tricaprylin, glycerol trioctanoate), with a saponification value of 335 mg KOH/g to 360 mg KOH/g e.g. commercially available Captex^{®} 8000, which is a synthetic triglyceride manufactured by esterification of caprylic acid and glycerin.
∘ caprylic/capric triglyceride with fatty-acid composition of 65% w/w to 80% w/w caprylic acid (C_{8:0}), 20% w/w to 35% w/w capric acid, less than 2% w/w caproic acid (C_{6:0}), less than 2% w/w lauric acid (C_{12:0}) and less than 1% w/w myristic acid (C_{14:0}), with a saponification value of 335 mg KOH/g to 355 mg KOH/g e.g. commercially available Miglyol^{®} 810, BergaBest^{®} MCT Oil 70/30 & Captex^{®} 300.
∘ caprylic/capric triglyceride with fatty-acid composition of 55% w/w to 70% w/w caprylic acid (C_{8:0}) & 30% w/w to 45% w/w capric acid (C_{10:0}), with a saponification value of 330 mg KOH/g to 360 mg KOH/g. e.g. commercially available Neobee^{®} M-5, Cromadol^{®} GTCC & Myritol^{®} 318.
∘ caprylic/capric triglyceride with fatty-acid composition of 50 w/w to 65% w/w caprylic acid (C_{8:0}), 30% w/w to 45% w/w capric acid (C_{10:0}), less than 2% w/w caproic acid (C_{6:0}), less than 2% w/w lauric acid (C_{12:0}) and less than 1% w/w myristic acid (C_{14:0}), with a saponification value of 325 mg KOH/g to 345 mg KOH/g e.g. commercially available Miglyol^{®} 812, BergaBest ^{®} MCT Oil 60/40 & Captex^{®} 355.

Preferably, the total concentration of the medium-chain triglycerides in the oral pharmaceutical suspension according to the invention is from 30% w/v to 85% w/v. More preferably, the total concentration of the medium-chain triglycerides is from 40% w/v to 80% w/v. Even more preferably, the total concentration of the medium-chain triglycerides is from 45% w/v to 75% w/v.

The oral pharmaceutical suspension of the invention provides excellent stability of capecitabine. This means that it is not necessary to include in the composition further stabilizing agents of capecitabine.

Thus, preferably, the oral pharmaceutical suspension according to the invention is free of any stabilizing agent which is not a medium chain triglyceride. For example, the oral pharmaceutical suspension according to the invention can be free of ethanol, propylene glycol, polyethylene glycol, sorbitol, glycerol, maltitol, polyvinylpyrrolidone, copolyvidone, sorbitan monolaurate, propylene glycol monolaurate (lauroglycol), as well as free of lipophilic surfactants such as polysorbate 80, polyethylene glycol castor oils and polyethylene glycol hydrogenated castor oils.

The oral pharmaceutical suspension, according to the invention may also comprise additional excipients commonly used in preparing oral liquid compositions, such as antimicrobial preservatives, antioxidants, sweeteners and flavouring agents.

Antimicrobial preservatives may include but are not limited to sodium benzoate, benzoic acid, boric acid, sorbic acid and their salts thereof, benzyl alcohol, parahydroxybenzoic acids and their alkyl esters, methyl, ethyl and propyl parahydroxybenzoates and their salts or mixtures thereof.

Antioxidants which may be used in the present invention comprise, amongst others, butylated hydroxytoluene, butylated hydroxyanisole, ethylenediamine tetraacetic acid ("EDTA"), ascorbic acid, sodium metabisulfite and propyl gallate or any combinations thereof.

Sweeteners may include but are not limited to aspartame, acesulfame potassium, thaumatin, saccharin and salts thereof, sodium cyclamate, glycyrrhizin, monosodium glycyrrhizinate, monoamonium glycyrrhizinate or mixtures thereof.

The oral pharmaceutical suspension, according to the invention may further comprise flavours and/or colours so as to enhance its palatability and/or visual appearance. Suitable flavouring agents and colouring agents are well known to those skilled in the art. The flavouring agent may be a natural or artificial flavouring agent, including an essence, an extract, a flavour oil or combinations thereof. Exemplary flavours include, but are not limited to: honey flavour, raspberry flavour, strawberry flavour, blueberry flavour, blackberry flavour, grape flavour, peach flavour, apricot flavour, watermelon flavour, melon flavour, fruit punch flavour, cranberry flavour, mango flavour, banana flavour, citrus flavour, orange flavour, lemon flavour, grapefruit flavour, cherry flavour, vanilla flavour, caramel flavour, chocolate flavour, marshmallow flavour, coffee flavour and coconut flavour.

The oral pharmaceutical suspension according to the invention is preferably supplied as multidose preparation. Each dose from a multidose container may be administered by means of a device suitable for accurately measuring the prescribed volume. The device is usually a spoon or a cup for volumes of 5 ml or multiples thereof, or an oral syringe for other volumes. Preferably, the device is an oral syringe.

The oral pharmaceutical suspension of the present invention may be prepared using methods well known in the art and using regular manufacturing equipment.

For example, it may be prepared using the following process: The active substance and the excipients are weighed. The selected medium-chain triglyceride(s) is added into a vessel and heated to 30 - 35°C. The selected suspending agent(s) is added into the vessel under continuous stirring. Capecitabine is added into the vessel under stirring. The remaining excipients if present, are successively added under continuous stirring. Finally, the volume is adjusted with a quantity of the medium-chain triglyceride(s).

The final suspension is optionally filtered over a 10 µm filter, and filled preferably in light-protective containers, such as amber type III glass 50 or 100 ml bottles sealed with child resistant, tamper evident screw caps.

### EXAMPLES

In the examples hereinbelow the terms "Impurity A", "Impurity B" and "Total impurities" have the following meanings:
Impurity A: 4-amino-1-(5-deoxy-β-D-ribofuranosyl)-5-fluoropyrimidin-2(1H)-one (5'-deoxy-5-fluorocytidine) according to Ph. Eur.- 10.0.
Impurity B: 1-(5-deoxy-β-D-ribofuranosyl)-5-fluoropyrimidine-2,4(1H,3H)-dione (5'-deoxy-5-fluorouridine) according to Ph. Eur.- 10.0.
The term "total impurities" refers to the sum of all capecitabine degradation impurities present in the oral suspension.

### EXAMPLE 1

The non-aqueous capecitabine suspensions of Table 1 (I, II, III & IV) were prepared in the following manner:
Approximately 80% of the total quantity of the selected medium-chain triglycerides, oils or glyceride mixtures was added into a vessel and heated to 30°C - 35°C. Capecitabine was added into the vessel under stirring. Colloidal silicon dioxide was then added into the vessel under continuous stirring. The remaining quantity of the selected medium-chain triglyceride, oils or glyceride mixtures were then added under continuous stirring. Finally, the volume was adjusted with the required quantity of the above medium-chain triglycerides, oils or glyceride mixtures.

Composition V of Table 1, disclosed in WO2020/039264, was prepared in the following manner:
Purified water was added into a (main) vessel. Glycerin was added into purified water under stirring. A pH buffer solution was prepared in a different vessel and was added under continuous stirring. Capecitabine was added into the main vessel under continuous stirring. Xanthan gum was also added into the vessel under stirring. The remaining excipients were successively added under continuous stirring. The pH of the suspension was adjusted with a quantity of the buffer solution to the desired value.

Finally, the volume was adjusted with purified water.

**Table 1**

| | | **Composition** | | | | |
|---|---|---|---|---|---|---|
| | **I** | **II** | **IIIa** | **IIIb** | **IV** | **V-aqueous (**WO 2020/039264**)** |
| **Active substance** | | **% w/v** | | | | |
| Capecitabine | 25 | 25 | 25 | 25 | 25 | 20 |

| **Excipients** | | **% w/v** | | | | |
|---|---|---|---|---|---|---|
| Crodamol^{™} GTCC (50 to 65% caprylic (C8:0) / 30% to 45% capric (C10:0) triglyceride) | 73 | 36.5 | 36.5 | 18.25 | | |
| Corn oil | - | 36.5 | - | - | - | - |
| Castor oil | - | - | 36.5 | 54.75 | | - |
| Glycerol dicaprylate | - | - | - | - | 73 | - |
| Colloidal silicon dioxide | 2 | 2 | 2 | 2 | 2 | - |
| Xanthan gum | - | - | - | - | - | 0.2 |
| Methylparaben | - | - | - | - | - | 0.07 |
| 30% simethicone emulsion | - | - | - | - | - | 0.5 |
| Sucralose | - | - | - | - | - | 0.2 |
| Tutti Frutti flavour | - | - | - | - | - | 0.02 |
| Glycerin | - | - | - | - | - | 58 |
| Citric acid monohydrate | - | - | - | - | - | 0.16 |
| Trisodium citrate dehydrate | - | - | - | - | - | 0.652 |
| Purified water | - | - | - | - | - | q.s. to 100 mL |

The compositions were stored at a temperature of 40°C and relative humidity of 75% for a period of three months. Quantification of capecitabine and its degradation impurities was performed by HPLC.

**Table 2**

| | **40°C± 2°C, 75% RH** | |
|---|---|---|
| | **T = 0** | **T = 3 months** |
| Trial I | Impurity A: 0.02% | Impurity A: 0.2% |
| | | Impurity B: 0.2% |
| | Impurity B: 0.03% | Unknown RRT=0.21: 0.01% |
| | Total: 0.1% | Total: 0.4% |
| Trial II | Impurity A: 0.03% | Impurity A: 0.2% |
| | | Impurity B: 0.3% |
| | Impurity B: 0.02% | Unknown RRT=0.21: 0.01% |
| | Total: 0.1% | Total: 0.5% |
| Trial Illa | Impurity A: 0.02% | Impurity A: 0.2% |
| | | Impurity B: 0.3% |
| | Impurity B: 0.04% | Unknown RRT=0.21: 0.01% |
| | Total: 0.1% | Total: 0.5% |
| Trial IIIb | Impurity A: 0.03% | Impurity A: 1.2% |
| | | Impurity B: 0.4% |
| | Impurity B: 0.05% | Unknown RRT=0.21: 0.02% |
| | Total: 0.1% | Total: 1.6% |
| Trial IV | Impurity A: 0.04% | Impurity A: 2.2% |
| | | Impurity B: 0.6% |
| | Impurity B: 0.02% | Unknown RRT=0.21: 0.02% |
| | Total: 0.1% | Total: 2.8% |
| Trial V | Impurity A: 0.03% | Impurity A: 4.4% |
| | | Impurity B: 1.8% |
| | Impurity B: 0.03% | Unknown RRT=0.21: 0.03% |
| | Total: 0.1% | Total: 6.2% |

The results of Table 2 above show that the inclusion of glycerol dicaprylate (diglyceride) to oral suspensions comprising capecitabine (composition IV), although seems to stabilize capecitabine to some extend compared to aqueous suspension V, it did not actually enhance the physicochemical stability of capecitabine at the desired level.

On the other hand, the above results clearly show that the physicochemical stability of capecitabine is considerably enhanced in non-aqueous liquid carriers comprising one or more medium-chain fatty acid triglycerides.

### EXAMPLE 2

These capecitabine suspensions were prepared in the following manner:
The active substance and the excipients were weighed. Approximately 80% of the total quantity of the selected medium-chain triglyceride(s) was added into a vessel and heated to 30°C - 35°C. The selected suspending agent was added into the vessel under continuous stirring. Capecitabine was added into the vessel under stirring. Finally, the volume was adjusted with the required quantity of the above medium-chain triglyceride(s).

In order to test the uniformity of the suspensions the following method was applied: A 65 ml bottle was filled with 60 ml of each of the prepared suspensions and stored for three months at room temperature (20°C to 30°C). After 10 seconds of hand shaking of the bottle, five 10 ml fractions were then carefully withdrawn from the surface of the liquid with a digital pipette and analyzed separately. The remaining 10 ml in the bottle, representing the sixth fraction, were also analyzed. Quantification of capecitabine in each of the six fractions was performed by HPLC. The results presented below show the range of capecitabine content in the six fractions as percentage of the label claim, i.e., of the theoretically calculated content of each fraction. Furthermore, the results show the relative standard deviation of the measurements.

**Table 3a:**

| | **Composition** | | | | |
|---|---|---|---|---|---|
| | **A** | **B** | **C** | **D** | **E** |
| **Active substance** | **% w/v** | | | | |
| Capecitabine | 25 | 25 | 25 | 25 | 25 |

| **Excipients** | **% w/v** | | | | |
|---|---|---|---|---|---|
| Miglyol 812^{®} | 73.5 | 73.5 | 73.5 | 73.5 | 73.5 |
| (50 to 65% caprylic (C_{8:0}) / 30% to 45% capric (C_{10:0}) triglyceride) | | | | | |
| Colloidal silicon dioxide | 1.5 | - | - | - | - |
| Bentonite | - | 1.5 | - | - | - |
| Potassium alginate | - | - | 1.5 | - | - |
| Xanthan gum | - | - | - | 1.5 | - |
| Vivapur^{®} MCG 591P | - | - | - | - | 1.5 |
| (Microcrystalline cellulose, carboxymethylcellulose sodium) | | | | | |
| Uniformity test results (90 days at room temperature) | | | | | |
| Range of six fractions (% label claim) | 97 - 102 | 86 - 116 | 89 - 113 | 90 - 109 | 94 - 104 |
| Relative Standard Deviation | 1.6% | 11.8% | 9.2% | 7.6% | 4.0% |

**Table 3b:**

| | **Composition** | | | | |
|---|---|---|---|---|---|
| | **A'** | **B'** | **C'** | **D'** | **E'** |
| **Active substance** | **% w/v** | | | | |
| Capecitabine | 25 | 25 | 25 | 25 | 25 |

| **Excipients** | **% w/v** | | | | |
|---|---|---|---|---|---|
| Glycerol monocaprylocaprate (Capmul MCM ^{®}) | 73.5 | 73.5 | 73.5 | 73.5 | 73.5 |
| Colloidal silicon dioxide | 1.5 | - | - | - | - |
| Bentonite | - | 1.5 | - | - | - |
| Potassium alginate | - | - | 1.5 | - | - |
| Xanthan gum | - | - | - | 1.5 | - |
| Vivapur^{®} MCG 591P (Microcrystalline cellulose / carboxymethylcellulose sodium) | - | - | - | - | 1.5 |

| Uniformity test results (90 days at room temperature) | | | | | |
|---|---|---|---|---|---|
| Range of six fractions (% label claim) | 90 - 109 | 82 - 119 | 85 - 116 | 88 - 110 | 90 - 108 |
| Relative Standard Deviation | 6.9% | 13.7% | 11.1% | 8.1% | 7.8% |

**Table 3c:**

| | **Composition** | |
|---|---|---|
| | F | **G** |
| **Active substance** | **% w/v** | |
| Capecitabine | 25 | 25 |

| **Excipients** | **% w/v** | |
|---|---|---|
| Miglyol 812^{®} | 73.5 | 73.5 |
| (50 to 65% caprylic (C_{8:0}) / 30% to 45% capric (C_{10:0}) triglyceride) | | |
| HPMC K4M (Hydroxypropyl methylcellulose) | 2.0 | - |
| HPC SL (Hydroxypropyl cellulose) | - | 1.5 |

| Uniformity test results (90 days at room temperature) | | |
|---|---|---|
| Range of six fractions (% label claim) | 69% (first fraction from surface of liquid*) | 70 - 128 |
| Relative Standard Deviation | Not measured | 23.7% |

| | | |
|---|---|---|
| *Only the first out of six factions withdrawn from the surface of the liquid with a digital pipette was analyzed. | | |

The uniformity test results of Tables 3a & 3b clearly show that the use of certain suspending agents (colloidal silicon dioxide, bentonite, potassium alginate, xanthan gum and microcrystalline cellulose / carboxymethylcellulose sodium) leads to acceptable redispersability of capecitabine particles in the oral suspension when the non-aqueous liquid carriers comprises either a medium chain triglyceride according to the invention (Table 3a) or the monoglyceride glycerol monocaprylocarpate (Table 3b). However, as explained in the following example, the use of glycerol monocaprylocarpate does not actually enhance the stability of capecitabine at the desired level.

Furthermore, the uniformity test results of table 3c show that the use of other suspending agents such as hydroxypropyl methylcellulose and hydroxypropyl cellulose does not maintain the content uniformity of capecitabine at desirable levels.

### EXAMPLE 3

The compositions of the present example do not belong to the present invention

The capecitabine suspensions of Table 4 were prepared in the following manner:
The active substance and the excipients were weighed. Approximately 80% of the total quantity of the selected medium-chain triglyceride(s) was added into a vessel and heated to 30°C - 35°C. The selected suspending agent was added into the vessel under continuous stirring. Capecitabine was added into the vessel under stirring. Finally, the volume was adjusted with the required quantity of the above medium-chain triglyceride(s).

**Table 4**

| | **Composition** | | |
|---|---|---|---|
| | **J** | **K** | **L** |
| **Active substance** | **% w/v** | | |
| Capecitabine | 25 | 25 | 25 |

| **Excipients** | **% w/v** | | |
|---|---|---|---|
| Glycerol monocaprylocarpate (Capmul MCM ^{®}) | 73 | 73 | 73 |
| Polysorbate 80 | 2 | - | - |
| Vivapur^{®} MCG 591P | - | 2 | - |
| (Microcrystalline cellulose / carboxymethylcellulose | | | |
| Colloidal silicon dioxide | | - | 2 |

The compositions were stored at a temperature of 40°C and relative humidity of 75% for a period of one or three months. Quantification of capecitabine and its degradation impurities was performed by HPLC.

**Table 5**

| | **40°C± 2°C, 75% RH** | |
|---|---|---|
| | **T = 0** | **T = 1 months** |
| Trial J | Impurity A: 0.04% | Impurity A: 0.6% |
| | Impurity B: 0.03% | Impurity B: 0.4% |
| | Total: 0.1% | Total: 1.0% |
| Trial K | Impurity A: 0.03% | Impurity A: 0.8% |
| | Impurity B: 0.03% | Impurity B: 0.5% |
| | Total: 0.1% | Total: 1.3% |

| | **T=0** | **T=3 months** |
|---|---|---|
| Trial L | Impurity A: 0.02% | Impurity A: 1.9% |
| | | Impurity B: 1.1% |
| | Impurity B: 0.04% | Unknown RRT=0.21: 0.02% |
| | Total: 0.1% | Total: 3.0% |

The results of table 5 show that the use of glycerol monocaprylocarpate does not actually enhance the stability of capecitabine at the desired level.

### EXAMPLE 4

Table 6 shows oral suspension compositions according to the present invention.

These capecitabine suspensions were prepared in the following manner:
The active substance and the excipients were weighed. Approximately 80% of the total quantity of the selected medium-chain triglyceride(s) was added into a vessel and heated to 30 - 35°C. Colloidal silicon dioxide was added into the vessel under stirring. Capecitabine was added into the vessel under stirring. The flavour was then added under continuous stirring. Finally, the volume was adjusted with the required quantity of the above medium-chain triglyceride(s).

**Table 6**

| | **Composition** | | |
|---|---|---|---|
| | **M** | **N** | **O** |
| **Active substance** | **% w/v** | | |
| Capecitabine | 25 | 25 | 25 |

| **Excipients** | **% w/v** | | |
|---|---|---|---|
| Miglyol 812 ^{®} (50 to 65% caprylic (C_{8:0}) / 30% to 45% capric (C_{10:0}) triglyceride) | 73.3 | 74.3 | 72.8 |
| Colloidal silicon dioxide | 1.5 | 0.5 | 2.0 |
| Mint flavour | 0.2 | 0.2 | 0.2 |

The compositions were stored at a temperature of 40°C and relative humidity of 75% for a period of three months. Quantification of capecitabine and its degradation impurities was performed by HPLC.

In order to test the uniformity of the suspensions the following method was applied: A 65 ml bottle was filled with 60 ml of each of the prepared oral suspensions and stored for three months at room temperature (20°C to 30°C). After 10 seconds of hand shaking of the bottle, five 10 ml fractions were then carefully withdrawn from the surface of the liquid with a digital pipette and analyzed separately. The remaining 10 ml in the bottle, representing the sixth fraction, was also analyzed. Quantification of capecitabine was performed by HPLC The results presented below show the range of capecitabine content in the six fractions as percentage of the label claim, i.e., of the theoretically calculated content of each fraction. Furthermore, the results show the relative standard deviation of the measurements.

**Table 7**

| | **Composition M** | **Composition N** | **Composition O** |
|---|---|---|---|
| **Appearance (T=0)** | White viscous suspension | White viscous suspension | White viscous suspension |
| **Assay (T= 0)** | 100.9% | 100.2% | 98.9% |
| **Impurity A (T= 0)** | 0.1% | 0.1% | 0.1% |
| **Impurity B (T= 0)** | 0.1% | 0.1% | 0.1% |
| **Total impurities (T= 0)** | 0.2% | 0.2% | 0.2% |
| **Appearance (T= 3 m)** | White viscous suspension | White viscous suspension | I White viscous suspension |
| **Assay (T= 3 m)** | 100.6% | 100.1% | 99.0% |
| **Impurity A (T= 3 m)** | 0.2% | 0.2% | 0.2% |
| **Impurity B (T= 3 m)** | 0.2% | 0.3% | 0.3% |
| **Total impurities (T= 3 m)** | 0.4% | 0.5% | 0.5% |

| Uniformity test results (90 days at room temperature) | | | |
|---|---|---|---|
| Range of six fractions (% label claim) | 97 - 102 | 98 - 102 | 96 - 104 |
| Relative Standard Deviation | 0.9% | 0.8% | 1.3% |

### EXAMPLE 5

Table 8 shows oral suspension compositions according to the present invention.

These capecitabine suspensions were prepared in the following manner:
The active substance and the excipients were weighed. Approximately 80% of the total quantity of the selected medium-chain triglyceride(s) was added into a vessel and heated to 30°C - 35°C. Capecitabine was added into the vessel under stirring.

The flavour was then added under continuous stirring. Finally, the volume was adjusted with the required quantity of the above medium-chain triglyceride(s).

**Table 8**

| | **Composition** | | | |
|---|---|---|---|---|
| | **P** | **Q** | **R** | **S** |
| **Active substance** | **% w/v** | | | |
| Capecitabine | 30 | 25 | 30 | 25 |

| **Excipients** | **% w/v** | | | |
|---|---|---|---|---|
| Miglyol 812 ^{®} | 67.3 | 36.15 | - | 36.15 |
| (50 to 65% caprylic (C_{8:0}) / 30% to 45% capric (C_{10:0}) triglyceride) | | | | |
| Miglyol 810 ^{®} | - | 36.15 | 67.3 | 36.15 |
| (65 to 80% caprylic (C_{8:0}) / 20% to 35% capric (C_{10:0}) triglyceride) | | | | |
| Colloidal silicon dioxide | 2.5 | 2.5 | 2.5 | 2.5 |
| Mint flavour | 0.2 | 0.2 | 0.2 | 0.2 |

The compositions were stored at a temperature of 40°C and relative humidity of 75% for a period of three months. Quantification of capecitabine and its degradation impurities was performed by HPLC.

**Table 9**

| | **Composition P** | **Composition Q** | **Composition R** | **Composition** S |
|---|---|---|---|---|
| **Appearance (T=0)** | White viscous suspension | White viscous suspension | White viscous suspension | White viscous suspension |
| **Assay (T= 0)** | 100.6% | 100.9% | 99.7% | 101.6% |
| **Impurity A (T= 0)** | 0.1% | 0.1% | 0.1% | 0.1% |
| **Impurity B (T= 0)** | 0.1% | 0.1% | 0.1% | 0.1% |
| **Total impurities (T= 0)** | 0.2% | 0.2% | 0.2% | 0.2% |
| **Appearance (T= 3 m)** | White viscous suspension | White viscous suspension | White viscous suspension | White viscous suspension |
| **Assay (T= 3 m)** | 99.8% | 100.0% | 98.9% | 99.8% |
| **Impurity A (T= 3 m)** | 0.2% | 0.2% | 0.4% | 0.2% |
| **Impurity B (T= 3m)** | 0.2% | 0.3% | 0.3% | 0.3% |
| **Total impurities (T= 3 m)** | 0.4% | 0.5% | 0.7% | 0.5% |

### EXAMPLE 6

Table 10 shows oral suspension compositions according to the present invention. These capecitabine suspensions were prepared as described in Example 5.

**Table 10**

| | **Composition** | | | |
|---|---|---|---|---|
| | **T** | **U** | **V** | **W** |
| **Active substance** | **% w/v** | | | |
| Capecitabine | 25 | 25 | 25 | 25 |

| **Excipients** | **% w/v** | | | |
|---|---|---|---|---|
| Captex 8000 ^{®} | 72.3 | 36.15 | - | 36.15 |
| (~99% caprylic (C_{8:0}) triglyceride) | | | | |
| Captex 355 ^{®} | - | 36.15 | 72.3 | 36.15 |
| (50% to 65% caprylic (C_{8:0}) / 30% to 45% capric (C_{10:0}) triglyceride) | | | | |
| Colloidal silicon dioxide | 2.5 | 2.5 | 2.5 | 2.5 |
| Tutti frutti flavour | 0.2 | 0.2 | 0.2 | 0.2 |

The compositions were stored at a temperature of 40°C and relative humidity of 75% for a period of three months. Quantification of capecitabine and its degradation impurities, in the compositions was performed by HPLC.

**Table 11**

| | **Composition T** | **Composition U** | **Composition V** | **Composition W** |
|---|---|---|---|---|
| **Appearance (T=0)** | White viscous suspension | White viscous suspension | White viscous suspension | White viscous suspension |
| **Assay (T= 0)** | 99.9% | 101.6% | 100.0% | 99.9% |
| **Impurity A (T= 0)** | 0.2% | 0.1% | 0.1% | 0.1% |
| **Impurity B (T= 0)** | 0.1% | 0.1% | 0.1% | 0.1% |
| **Total impurities (T= 0)** | 0.2% | 0.2% | 0.2% | 0.2% |
| **Appearance (T= 3 m)** | White viscous suspension | White viscous suspension | White viscous suspension | White viscous suspension |
| **Assay (T= 3 m)** | 99.1% | 100.7% | 99.4% | 99.1% |
| **Impurity A (T= 3 m)** | 0.3% | 0.3% | 0.3% | 0.4% |
| **Impurity B (T= 3m)** | 0.3% | 0.2% | 0.3% | 0.4% |
| **Total impurities (T= 3 m)** | 0.6% | 0.5% | 0.6% | 0.8% |

## Claims

1. Oral pharmaceutical suspension comprising capecitabine as active ingredient and a non-aqueous liquid carrier comprising one or more triglycerides of saturated fatty acid having a straight chain of 6 to 10 carbon atoms (medium-chain triglycerides) and at least one suspending agent selected form the group consisting of colloidal silicon dioxide, bentonite, potassium alginate, xanthan gum and a mixture of microcrystalline cellulose and sodium carboxymethylcellulose.

2. Oral pharmaceutical suspension according to claim 1, comprising from 5.0% w/v to 40% w/v capecitabine.

3. Oral pharmaceutical suspension according to claim 1 or 2, comprising from 10% w/v to 30% w/v capecitabine.

4. Oral pharmaceutical suspension according to any one of the preceding claims, wherein the total concentration of medium-chain triglycerides in the suspension is from 30% w/v to 85% w/v.

5. Oral pharmaceutical suspension according to any one of the preceding claims, wherein the total concentration of medium-chain triglycerides in the suspension is from 40% w/v to 80% w/v.

6. Oral pharmaceutical suspension according to any one of the preceding claims, wherein the total concentration of medium-chain triglycerides in the suspension is from 45% w/v to 75% w/v.

7. Oral pharmaceutical suspension according to any one of the preceding claims, wherein the one or more medium-chain triglycerides is caprylic triglyceride.

8. Oral pharmaceutical suspension according to any one of the preceding claims, wherein the one or more medium-chain triglycerides is a mixture of caprylic and capric triglycerides in a weight ratio caprylic acid : capric acid from 4:6 to 99:1.

9. Oral pharmaceutical suspension according to any one of the preceding claims, wherein the one or more suspending agents are selected from the group consisting of colloidal silicon dioxide, potassium alginate and a mixture of microcrystalline cellulose and sodium carboxymethylcellulose.

10. Oral pharmaceutical suspension according to any one of the preceding claims, wherein the one or more suspending agents is colloidal silicon dioxide.

11. Oral pharmaceutical suspension according to any one of the preceding claims, wherein the total concentration of suspending agents in the suspension is from 0.1% w/v to 10% w/v.

12. Oral pharmaceutical suspension according to any one of the preceding claims, wherein the total concentration of suspending agents in the suspension is from 1% w/v to 5% w/v.

13. Oral pharmaceutical suspension according to any one of the preceding claims, wherein the one or more medium-chain triglycerides have a saponification value from 320 mg KOH/g to 380 mg KOH/g.

14. Oral pharmaceutical suspension according to any one of the preceding claims, wherein the suspension is free of ethanol, propylene glycol, polyethylene glycol, sorbitol, glycerol, maltitol, polyvinylpyrrolidone, copolyvidone, sorbitan monolaurate, propylene glycol monolaurate (lauroglycol), and lipophilic surfactants such as polysorbate 80, polyethylene glycol castor oils and polyethylene glycol hydrogenated castor oils.

15. Oral pharmaceutical suspension according to any one of the preceding claims, wherein the suspension is free of any stabilizing agent which is not a medium chain triglyceride.

## Patentansprüche

1. Orale pharmazeutische Suspension, umfassend Capecitabin als Wirkstoff und einen nichtwässrigen flüssigen Träger, der ein oder mehrere Triglyceride gesättigter Fettsäuren mit einer geraden Kette von 6 bis 10 Kohlenstoffatomen (mittelkettige Triglyceride) und mindestens ein Suspensionsmittel umfasst, das aus der Gruppe ausgewählt ist, die aus kolloidalem Siliciumdioxid, Bentonit, Kaliumalginat, Xanthangummi und einer Mischung aus mikrokristalliner Cellulose und Natriumcarboxymethylcellulose besteht.

2. Orale pharmazeutische Suspension nach Anspruch 1, umfassend 5,0 % w/v bis 40 % w/v Capecitabin.

3. Orale pharmazeutische Suspension nach Anspruch 1 oder 2, umfassend 10 % w/v bis 30 % w/v Capecitabin.

4. Orale pharmazeutische Suspension nach einem der vorstehenden Ansprüche, wobei die Gesamtkonzentration an mittelkettigen Triglyceriden in der Suspension 30 % w/v bis 85 % w/v beträgt.

5. Orale pharmazeutische Suspension nach einem der vorstehenden Ansprüche, wobei die Gesamtkonzentration an mittelkettigen Triglyceriden in der Suspension 40 % w/v bis 80 % w/v beträgt.

6. Orale pharmazeutische Suspension nach einem der vorstehenden Ansprüche, wobei die Gesamtkonzentration an mittelkettigen Triglyceriden in der Suspension 45 % w/v bis 75 % w/v beträgt.

7. Orale pharmazeutische Suspension nach einem der vorstehenden Ansprüche, wobei es sich bei dem einen oder den mehreren mittelkettigen Triglyceriden um Caprylsäuretriglycerid handelt.

8. Orale pharmazeutische Suspension nach einem der vorstehenden Ansprüche, wobei es sich bei dem einen oder den mehreren mittelkettigen Triglyceriden um eine Mischung aus Caprylsäure- und Caprinsäuretriglyceriden in einem Gewichtsverhältnis Caprylsäure: Caprinsäure von 4:6 bis 99:1 handelt.

9. Orale pharmazeutische Suspension nach einem der vorstehenden Ansprüche, wobei das eine oder die mehreren Suspensionsmittel aus der Gruppe ausgewählt sind, die aus kolloidalem Siliciumdioxid, Kaliumalginat und einer Mischung aus mikrokristalliner Cellulose und Natriumcarboxymethylcellulose besteht.

10. Orale pharmazeutische Suspension nach einem der vorstehenden Ansprüche, wobei es sich bei dem einen oder den mehreren Suspensionsmitteln um kolloidales Siliciumdioxid handelt.

11. Orale pharmazeutische Suspension nach einem der vorstehenden Ansprüche, wobei die Gesamtkonzentration an Suspensionsmitteln in der Suspension 0,1 % w/v bis 10 % w/v beträgt.

12. Orale pharmazeutische Suspension nach einem der vorstehenden Ansprüche, wobei die Gesamtkonzentration an Suspensionsmitteln in der Suspension 1 % w/v bis 5 % w/v beträgt.

13. Orale pharmazeutische Suspension nach einem der vorstehenden Ansprüche, wobei das eine oder die mehreren mittelkettigen Triglyceride eine Verseifungszahl von 320 mg KOH/g bis 380 mg KOH/g aufweisen.

14. Orale pharmazeutische Suspension nach einem der vorstehenden Ansprüche, wobei die Suspension frei von Ethanol, Propylenglykol, Polyethylenglykol, Sorbit, Glycerin, Maltit, Polyvinylpyrrolidon, Copolyvidon, Sorbitanmonolaurat, Propylenglykolmonolaurat (Lauroglycol) und lipophilen Tensiden wie Polysorbat 80, Polyethylenglykol-Rizinusölen und hydrierten Polyethylenglykol-Rizinusölen ist.

15. Orale pharmazeutische Suspension nach einem der vorstehenden Ansprüche, wobei die Suspension frei von jeglichem Stabilisierungsmittel ist, das kein mittelkettiges Triglycerid ist.

## Revendications

1. Suspension pharmaceutique orale comprenant de la capécitabine comme ingrédient actif et un support liquide non aqueux comprenant un ou plusieurs triglycérides d'acide gras saturé ayant une chaîne linéaire de 6 à 10 atomes de carbone (triglycérides de chaîne moyenne) et au moins un agent de suspension choisi dans le groupe constitué par le dioxyde de silicium colloïdal, la bentonite, l'alginate de potassium, la gomme de xanthane et un mélange de cellulose microcristalline et de carboxyméthylcellulose de sodium.

2. Suspension pharmaceutique orale selon la revendication 1, comprenant de 5,0 % p/v à 40 % p/v de capécitabine.

3. Suspension pharmaceutique orale selon la revendication 1 ou 2, comprenant de 10 % p/v à 30 % p/v de capécitabine.

4. Suspension pharmaceutique orale selon l'une quelconque des revendications précédentes, dans laquelle la concentration totale de triglycérides de chaîne moyenne dans la suspension est de 30 % p/v à 85 % p/v.

5. Suspension pharmaceutique orale selon l'une quelconque des revendications précédentes, dans laquelle la concentration totale de triglycérides de chaîne moyenne dans la suspension est de 40 % p/v à 80 % p/v.

6. Suspension pharmaceutique orale selon l'une quelconque des revendications précédentes, dans laquelle la concentration totale de triglycérides de chaîne moyenne dans la suspension est de 45 % p/v à 75 % p/v.

7. Suspension pharmaceutique orale selon l'une quelconque des revendications précédentes, dans laquelle l'un ou plusieurs triglycérides de chaîne moyenne est le triglycéride caprylique.

8. Suspension pharmaceutique orale selon l'une quelconque des revendications précédentes, dans laquelle l'un ou plusieurs triglycérides de chaîne moyenne est un mélange de triglycérides caprylique et caprique dans un rapport en poids acide caprylique : acide caprique de 4:6 à 99:1.

9. Suspension pharmaceutique orale selon l'une quelconque des revendications précédentes, dans laquelle l'un ou plusieurs agents de suspension sont choisis dans le groupe constitué par le dioxyde de silicium colloïdal, l'alginate de potassium et un mélange de cellulose microcristalline et de carbooxyméthylcellulose de sodium.

10. Suspension pharmaceutique orale selon l'une quelconque des revendications précédentes, dans laquelle l'un ou plusieurs agents de suspension est le dioxyde de silicium colloïdal.

11. Suspension pharmaceutique orale selon l'une quelconque des revendications précédentes, dans laquelle la concentration totale des agents de suspension dans la suspension est de 0,1 % p/v à 10 % p/v.

12. Suspension pharmaceutique orale selon l'une quelconque des revendications précédentes, dans laquelle la concentration totale des agents de suspension dans la suspension est de 1 % p/v à 5 % p/v.

13. Suspension pharmaceutique orale selon l'une quelconque des revendications précédentes, dans laquelle l'un ou plusieurs triglycérides de chaîne moyenne ont une valeur de saponification de 320 mg KOH/g à 380 mg KOH/g.

14. Suspension pharmaceutique orale selon l'une quelconque des revendications précédentes, dans laquelle la suspension est exempte d'éthanol, de propylène glycol, de polyéthylène glycol, de sorbitol, de glycérol, de maltitol, de polyvinylpyrrolidone, de copolyvidone, de monolaurate de sorbitan, de monolaurate de propylène glycol (lauroglycol) et de tensioactifs lipophiles tels que le polysorbate 80, les huiles de ricin de polyéthylène glycol et les huiles de ricin hydrogénées de polyéthylène glycol.

15. Suspension pharmaceutique orale selon l'une quelconque des revendications précédentes, dans laquelle la suspension est exempte de tout agent stabilisant qui n'est pas un triglycéride de chaîne moyenne.
